(19) Europäisches Patentamt
European Patent Office
Office européen des brevets

(11) Veröffentlichungsnummer: **0 170 117**
**B1**

# (12) EUROPÄISCHE PATENTSCHRIFT

(45) Veröffentlichungstag der Patentschrift: **30.01.91**

(51) Int. Cl.$^5$: **C 07 D 209/34**, A 61 K 31/40

(21) Anmeldenummer: **85108579.5**

(22) Anmeldetag: **10.07.85**

## (54) Oxindol-Derivate, Verfahren zu ihrer Herstellung, diese Verbindungen enthaltende Arzneimittel und Zwischenprodukte.

(30) Priorität: **18.07.84 DE 3426419**

(43) Veröffentlichungstag der Anmeldung:
**05.02.86 Patentblatt 86/06**

(45) Bekanntmachung des Hinweises auf die Patenterteilung:
**30.01.91 Patentblatt 91/05**

(84) Benannte Vertragsstaaten:
**AT BE CH DE FR GB IT LI LU NL SE**

(56) Entgegenhaltungen:
**EP-A-0 004 532**
**EP-A-0 005 828**
**EP-A-0 121 176**
**WO-A-85/02550**
**FR-A-2 143 343**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 116**
**D-6800 Mannheim 31 (DE)**

(72) Erfinder: **Michel, Helmut**
**Ziegelgasse 2a**
**D-6800 Mannheim 31 (DE)**
Erfinder: **Kampe, Wolfgang, Dr. rer. nat.**
**Zedernstrasse 49**
**D-6805 Heddesheim (DE)**
Erfinder: **Strein, Klaus, Prof.**
**Eichenstrasse 45**
**D-6944 Hemsbach (DE)**
Erfinder: **Bartsch, Wolfgang, Dr. med. vet.**
**Franconviller-Strasse 5**
**D-6806 Viernheim (DE)**

**EP 0 170 117 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Oxindol-Derivate der allgemeinen Formel I

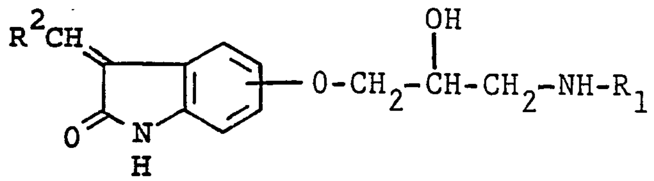

(I)

in welcher

$R_1$ eine $C_2$—$C_{10}$-Nitratoalkylgruppe,

$R_2$ einen geradkettigen oder verzweigten $C_1$—$C_6$-Alkylrest, einen Cyclopentyl- oder Cyclohexylrest, oder eine unsubstituierte oder durch Halogen-, $C_1$—$C_6$-Alkyl, $C_1$—$C_6$-Hydroxyalkyl-, Hydroxy-, $C_1$—$C_6$-Alkoxy-, $C_2$—$C_4$-Alkenyl-, $C_2$—$C_4$-Alkenyloxy, $C_2$—$C_4$-Alkinyl-, Amino-, $C_1$—$C_4$-Alkylamino-, $C_2$—$C_8$-Dialkylamino-, Aminocarbonyl-, $C_1$—$C_4$-Alkylaminocarbonyl-, $C_2$—$C_8$-Dialkylaminocarbonyl-, Cyano-, $C_2$—$C_4$-Alkanoyl-, Aminosulfonyl-, $C_1$—$C_4$-Alkylaminosulfonyl-, $C_2$—$C_8$-Dialkylaminosulfonyl-, Carboxyl-, $C_1$—$C_6$-Alkoxycarbonyl-, $C_1$—$C_6$-Alkylthio-, $C_1$-$C_6$-Alkylsulfinyl-, $C_1$—$C_6$-Alkylsulfonyl-, $C_2$—$C_4$-Alkanoylamido-, $C_1$—$C_4$-Alkylsulfonylamido- oder Nitrogruppen oder durch eine $C_1$—$C_2$-Alkylendioxygruppe substituierte Phenyl-, Furyl-, Thiophenyl, Pyrrolyl-, Pyrazolyl-, Imidazolyl-, Triazolyl-, Tetrazolyl-, Oxoimidazolinyl-, Pyridinyl-, Pyrimidinyl-, Uracilyl-, Indolyl-, Indazolyl- oder Dihydropyranylgruppe darstellt, deren optisch aktive Formen sowie deren pharmakologisch unbedenkliche Salze.

Gegenstand der Erfindung sind ferner pharmakologisch vertraegliche Salze der beanspruchten Verbindungen, Verfahren zu ihrer Herstellung und diese Verbindungen enthaltende Arzneimittel und Zwischenprodukte.

Da die Verbindungen der allgemeinen Formel I ein asymmetrisches Kohlenstoffatom besitzen, sind ferner Gegenstand der Erfindung auch die optisch aktiven Formen und racemische Gemische dieser Verbindungen.

Deren E- und Z-Isomere Gegenstand der Erfindung.

Die erfindungsgemaeßen Verbindungen sowie ihre pharmakologisch unbedenklichen Salze zeigen im gleichen Dosisbereich nitratartige sowie β-blockierende Wirkungen und eignen sich daher zur Behandlung und Prophylaxe bei Kreislauf- und Herzerkrankungen, wie z.B. Hochdruck und Angina pectoris.

Die französische Patentanmeldung FR—A—2,143,343 beschreibt Oxindol-Derivate, die in 4- bis 7-Stellung des Oxindolrings durch die Aminopropanolgruppe —O—$CH_2$—$CH(OR_3)$—$CH_2$—$NHR_2$ substituiert sind. Diese Verbindungen unterscheiden sich von den erfindungsgemäßen Verbindungen einerseits dadurch, daß der Substituent $R_2$ in keinem Fall eine Nitratogruppe enthalten kann, und andererseits dadurch, daß dort nur in 3-Stellung unsubstituierte Oxindole beschrieben sind.

Bei den aus EP—A—5,828 bekannten Verbindungen handelt es sich um Oxindol- und Chinolinon-Derivate, bei denen die Aminkomponenten der Aminopropanolgruppe einen Piperazinring darstellt, der durch eine Phenylgruppe substituiert ist. Auch diese Verbindungen enthalten keine Nitratogruppe.

Aus EP—A—121,176 sind in 4-Stellung durch die Aminopropanolgruppe —O—$CH_2$—CHOH—$CH_2$—NH—$R_1$ substituierte Oxindole bekannt, wobei $R_1$ eine Alkyl- oder Phenoxyalkyl- bzw. Phenylthioalkylgruppe bedeutet. Auch in diesem Fall kommt die Nitratogruppe als Substituent der Gruppe $R_1$ nicht in Frage.

Strukturell nahestehende Verbindungen sind in der EP—B—4,532 beschrieben, die bezüglich der Aminopropanolkette auch durch Nitratogruppen substituierte Derivate offenbart. Unter anderem wird dort als aromatischer Rest Ar in der Definition von Ar —O—$CH_2$—CHOH—$CH_2$—NH—R auch die Indolylgruppe beschrieben, die durch Alkylrest substituiert sein kann.

Die $C_2$—$C_{10}$-Nitratoalkylgruppen des Substituenten $R_1$ sind geradkettige oder verzweigte Gruppen wie Nitratoethyl-, Nitratopropyl-, Nitratobutyl-, Nitratopentyl-, Nitratohexyl-, 1-Methyl-2-nitraoethyl-, 1-Methyl-3-nitratopropyl-, 1,1-Dimethyl-3-nitratopropyl- 1,3-Dimethyl-3-nitratopropyl-, 2,2-Dimethyl-3-nitratopropyl-Gruppen.

Insbesondere kommen die 1-Methyl-3-nitratopropyl-, 1,1-Dimethyl-3-nitratopropyl- und die 1,3-Dimethylnitratopropylgruppe in Frage.

Unter $C_1$—$C_6$-Alkylengruppen des Substituenten $R_2$ sind geradkettige oder verzweigte Gruppen wie z.B. Methyl-, Ethyl-, Propyl-, Isopropyl-, n-Butyl-, Isobutyl-, tert. Butyl- oder n-Hexylgruppen zu verstehen. Bevorzugt sind jedoch die Methyl-, Ethyl-, Isopropyl- und tert. Butylgruppe.

Die als Substituenten des Phenylrestes bzw. der Hetarylreste in Frage kommenden Alkyl-, Alkoxy-, Alkylthio-, Alkylsulfinyl-, Alkylsulfonyl-, Alkoxycarbonyl-, Alkylaminocarbonyl- bzw. Dialkylaminocarbonyl-, Alkylaminosulfonyl- bzw. Dialkylaminosulfonyl-, Alkanoyl-, Alkylsulfonylamido-, Dialkyl-amino- berichalten bevorzugt den Methyl- und Ethylrest sowie die verschiedenen isomeren Propyl-, Butyl- und gegebenenfalls Pentylgruppen.

Bei den Alkenyl- und Alkinylgruppen kommen insbesondere die Allyl- und Propargylgruppe in Frage.

Unter Halogen wird in Sinne der Erfindung Fluor, Chlor, Brom und Jod verstanden, vorzugsweise Fluor, Chlor und Brom.

Von dem Hetaryl-Resten $R_2$ sind der Furanyl-, Thiophenyl-, Pyrrolyl-, Pyrazolyl-, Imidazolyl-, Triazolyl-, Tetrazolyl-, Oxoimidazolinyl-, Pyridinyl-, Pyrimidinyl-, Uracilyl-, Indolyl-, Indazolyl- und Dihydropyranyl-Rest bevorzugt.

Die Herstellung der Verbindungen der allgemeinen Formel I ist dadurch gekennzeichnet, daß man in an sich bekannter Weise entweder

a) eine Verbindung der allgemeinen Formel II

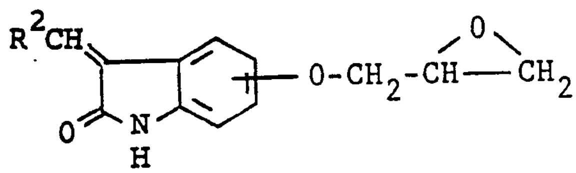

(II)

mit einer Verbindung der allgemeinen Formel III

$$H_2N—R_1 \qquad (III),$$

wobei $R_1$ und $R^2$ die oben angegebene Bedeutung haben, umsetzt oder

b) eine Verbindung der allgemeinen Formel IIa

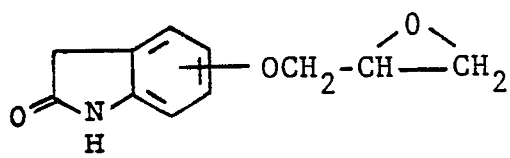

(IIa)

mit einer Verbindung der allgemeinen Formel IV

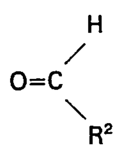

(IV),

in welcher $R^2$ die oben angegebenen Bedeutung hat kondensiert und anschließend mit einer Verbindung der allgemeinen Formel III
umsetzt oder

c) eine Verbindung der allgemeinen Formel V

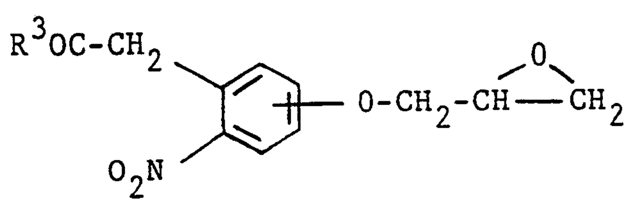

(V)

in welcher $R_6$ eine abspaltbare Gruppe darstellt, reduziert, mit einer Verbindung der allgemeinen Formel III unsetzt und
die dabei enthaltene Verbindung der allgemeinen Formel VI

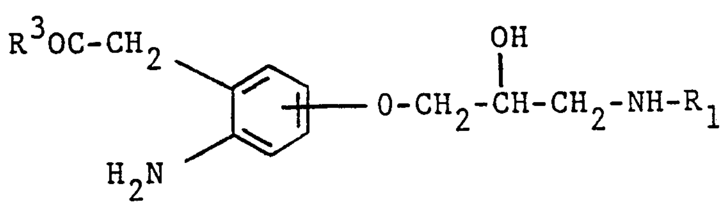

(VI)

in welcher $R_1$ und $R_3$ die oben angegebenen Bedeutung haben, cyclisiert oder

d) eine Verbindung der allgemeinen Formel VII

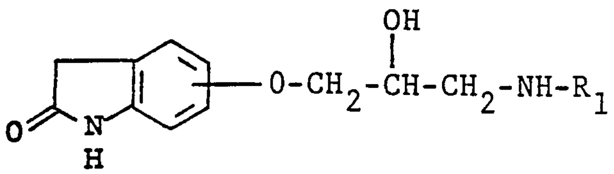

(VII)

in welcher $R_1$, die oben angegebene Bedeutung hat, mit einer Verbindung der allgemeinen Formel IV umsetzt oder

e) eine Verbindung der allgemeinen Formel VIII

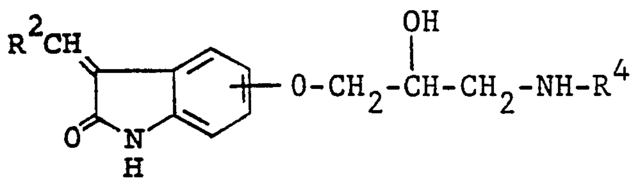

(VIII)

in welcher $R^2$ die oben angegebene Bedeutung hat, und $R^4$ eine $C_2$—$C_{10}$ Hydroxyalkylgruppe darstellt, mit Salpetersäure oder einem reaktiven Derivat davon, umsetzt, und gewünschtenfalls die erhaltenen Verbindungen in ein verträgliches Salz überführt.

Die Herstellung der Verbindungen der Formel II sowie einige Vertreter, insbesondere die 4-substituierten, ist in der DE—A—33 10 891.1 beschrieben. Neue Verbindungen koennen analog hergestellt werden.

Verbindungen der allgemeinen Formel III und IV werden nach literaturbekannten Verfahren hergestellt bzw. sind käuflich.

Verbindungen der allgemeinen Formel V sind teilweise in der EP—B1 00 149 28 beschrieben. Die neuen Verbindungen können analog hergestellt werden.

Verbindungen der Formel VI sind neu. Gegenstand der Erfindung sind demnach auch neue Zwischenprodukte der allgemeinen Formel VI zur Herstellung von Verbindungen der Formel I.

Abspaltbare Gruppen der Formeln V und VI sind Amino-, Imidazolyl-, Hydroxy- oder $C_1$—$C_6$-Alkoxygruppen, vorzugsweise Hydroxy-, Methoxy-, Ethoxy- und Propoxy-gruppen.

Die Umsetzungen der Verbindungen der allgemeinen Formel VII mit Verbindungen der allgemeinen Formel IV koennen ohne Loesungsmittel oder in einem inerten Loesungsmittel, wie z.B. Methanol, Ethanol, n-Butanol, Diethylether, Methylenchlorid, Toluol, Essigester, Terahydrofuran, Dioxan, Dimethylformamid oder Dimethylsulfoxid unter Zusatz eines geeigneten Katalysators, wie z.B. Ammoniak, Triethylamin, N-Ethyl-diisopropylamin, Tributylamin, Piperidin, Morpholin, 1-Methylpiperidin, 4-Methylmorpholin oder Natriummethylat durchgefuehrt werden. Besonders geeignet sind jedoch Methanol, Ethanol und Dimethylsulfoxid sowie Triethylamin, Piperidin und 1-Methylpiperidin.

Verbindungen der Formel VII sind per se pharmakologisch wirksam, können aber auch als Zwischenprodukte zur Herstellung von anderen wirksamen Verbindungen der Formel I verwendet werden.

Die erfindungsgemaeßen Verbindungen der Formel I koennen in Form eines racemischen Gemisches anfallen. Die Trennung des Racemats in die optisch aktiven Formen geschieht nach an sich bekannten Methoden ueber die diastereomeren Salze aktiver Saeuren, wie z.B. Weinsaeure, Aepfelsaeure oder Camphersulfonsaeure.

Verbindungen der Formel VIII und VIIIa sind ebenfalls neu und auch Gegenstand der Erfindung. Die Herstellung dieser Verbindungen geschieht vorzugsweise durch Umsetzung von Epoxyden der allgemeinen Formel II oder V mit einer Verbindung der allgemeinen Formel IX.

$$H_2N—R_5 \qquad (IX)$$

in der $R_5$ die oben genannte Bedeutung hat.

Die Umsetzung mit Salpetersäure bzw. deren reaktiven Derivate, wie Nitroniumtetrafluoborat, geschieht vorzugsweise in Acetonitril.

Zur Ueberfuehrung der Verbindungen der allgemeinen Formel I in ihre pharmakologisch unbedenklichen Salze setzt man diese, vorzugsweise in einem organischen Loesungsmittel, mit der aequivalenten Menge einer anorganischen oder organischen Saeure, z.B. Salzsaeure, Bromwasserstoffsaeure, Phosphorsaeure, Schwefelsaeure, Essigsaeure, Citronensaeure, Weinsaeure, Maleinsaeure, Fumarsaeure, Benzosaeure oder Cyclohexylsulfaminsaeure um.

Zur Herstellung von Arzneimittelnwerden die Substanzen der allgemeinen Formel I in an sich

bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Oliven-oel, suspendiert oder geloest.

Die erfindungsgemaßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nichttoxische Salze), hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Bevorzugt im Sinne der vorliegenden Anmeldung sind außer den in den Beispielen genannten Verbindungen die folgenden;

4-[2-Hydroxy-3-(1-methyl-3-nitratopropylamino)propoxy]-3-(3-chlor-benzyliden)indolinon-fumarat, Fp. 97—100°C aus Propanol-2, Ausb. 30%

4-[2-Hydroxy-3-(1-methyl-3-nitratopropylamino)propoxy]-3-(2-fluor-benzyliden)indolinon

4-[2-Hydroxy-3-(1-methyl-3-nitratopropylamino)propoxy]-3-(2-methyl-benzyliden)indolinon-fumarat, Fp. 80—85°C aus Propanol-2, Ausb. 20%

4-[2-Hydroxy-3-(1-methyl-3-nitratopropylamino)propoxy]-3-(2-hydroxymethyl-benzyliden)indolinon

4-[2-Hydroxy-3-(1-methyl-3-nitratopropylamino)propoxy]-3-(4-hydroxy-benzyliden)indolinon

4-[2-Hydroxy-3-(1-methyl-3-nitratopropylamino)propoxy]-3-(3-methoxy-benzyliden)indolinon-acetat, Fp. 80—83°C aus Essigester, Ausb. 25%

4-[2-Hydroxy-3-(1-methyl-3-nitratopropylamino)propoxy]-3-(2-allyloxy-benzyliden)indolinon

4-[2-Hydroxy-3-(1-methyl-3-nitratopropylamino)propoxy]-3-(4-amino-benzyliden)indolinon

4-[2-Hydroxy-3-(1-methyl-3-nitratopropylamino)propoxy]-3-(4-dimethylamino-benzyliden)indolinon

4-[2-Hydroxy-3-(1-methyl-3-nitratopropylamino)propoxy]-3-(2-acetamido-benzyliden)indolinon

4-[2-Hydroxy-3-(1-methyl-3-nitratopropylamino)propoxy]-3-(2-methansulfonylamido-benzyliden)-indolinon

4-[2-Hydroxy-3-(1-methyl-3-nitratopropylamino)propoxy]-3-(4-acetyl-benzyliden)indolinon

4-[2-Hydroxy-3-(1-methyl-3-nitratopropylamino)propoxy]-3-(4-aminocarbonyl-benzyliden)indolinon

4-[2-Hydroxy-3-(1-methyl-3-nitratopropylamino)propoxy]-3-(4-dimethylaminocarbonyl-benzyliden)-indolinon

4-[2-Hydroxy-3-(1-methyl-3-nitratopropylamino)propoxy]-3-(2-carboxy-benzyliden)indolinon

4-[2-Hydroxy-3-(1-methyl-3-nitratopropylamino)propoxy]-3-(4-methoxycarbonyl-benzyliden)indolinon

4-[2-Hydroxy-3-(1-methyl-3-nitratopropylamino)propoxy]-3-(4-nitro-benzyliden)indolinon, Fp. 135—137°C aus Methanol, Ausb. 30%

4-[2-Hydroxy-3-(1-methyl-3-nitratopropylamino)propoxy]-3-(2-dimethylaminosulfonyl-benzyliden)-indolinon

4-[2-Hydroxy-3-(1-methyl-3-nitratopropylamino)propoxy]-3-(3-methoxy-4-hydroxy-benzyliden)-indolinon

4-[2-Hydroxy-3-(1-methyl-3-nitratopropylamino)propoxy]-3-(2-hydroxy-4-methylmercapto-benzyliden)indolinon

4-[2-Hydroxy-3-(1-methyl-3-nitratopropylamino)propoxy]-3-(3,4-methylendioxy-benzyliden)indolinon

4-[2-Hydroxy-3-(1-methyl-3-nitratopropylamino)propoxy]-3-(2-Methylaminosulfonyl-furan-5-yl)-methylen-indolinon

4-[2-Hydroxy-3-(1-methyl-3-nitratopropylamino)propoxy]-3-(4-methylimidazolin-2-on-5-yl)methylen-indolinon

4-[2-Hydroxy-3-(1-methyl-3-nitratopropylamino)propoxy]-3-(pyridin-4-yl)methylen-indolinon

4-[2-Hydroxy-3-(1-methyl-3-nitratopropylamino)propoxy]-3-(5,6-dihydro-2H-pyran-3-yl)methylen-indolinon

4-[2-Hydroxy-3-(1-methyl-3-nitratopropylamino)propoxy]-3-(indol-3-yl)methylen-indolinon

5-[2-Hydroxy-3-(1-methyl-3-nitratopropylamino)propoxy]-3-(2-methylpropyliden)indolinon

5-[2-Hydroxy-3-(1-methyl-3-nitratopropylamino)propoxy]-3-(cyclohexylmethylen)-indolinon

4-[2-Hydroxy-3-(1-methyl-3-nitratopropylamino)propoxy]-3-(3-methyl-pyrazol-5-yl)methylen-indolinon Fp. 127—129°C aus Essigester; Ausb. 25%

Bevorzugte Zwischenprodukte der allgemeinen Formel VI sind:

2-[2-Hydroxy-3-(1-methyl-3-nitratopropylamino)propoxy]-6-amino-phenylessigsäure,

2-[2-Hydroxy-3-(1,1-dimethyl-3-nitratopropylamino)propoxy]-6-amino-phenylessigsäure

2-[2-Hydroxy-3-(1,3-dimethyl-3-nitratopropylamino)propoxy]-6-amino-phenylessigsäure

2-[2-Hydroxy-3-(2,2-dimethyl-3-nitratopropylamino)propoxy]-6-amino-phenylessigsäure

sowie deren Imidazolide, Methyl- und Ethylester und andere reaktive Derivate.

Bevorzugte Zwischenprodukte gemäß Formel VIII sind außer den in den Beispielen genannten folgenden Verbindungen:

5-[2-Hydroxy-3-(1-methyl-3-hydroxypropylamino)propoxy]-indolinon
4-[2-Hydroxy-3-(1,1-dimethyl-3-hydroxypropylamino)propoxy]-indolinon
4-[2-Hydroxy-3-(1,3-dimethyl-3-hydroxypropylamino)propoxy]-indolinon
4-[2-Hydroxy-3-(2,2-dimethyl-3-hydroxypropylamino)propoxy]-indolinon
4-[2-Hydroxy-3-(1-methyl-3-hydroxypropylamino)propoxy]-3-methyl-indolinon
4-[2-Hydroxy-3-(1,1-dimethyl-3-hydroxypropylamino)propoxy]-3-methyl-indolinon
5-[2-Hydroxy-3-(1,1-dimethyl-3-hydroxypropylamino)propoxy]-3-methyl-indolinon
4-[2-Hydroxy-3-(1,3-dimethyl-3-hydroxypropylamino)propoxy]-3-methyl-indolinon
4-[2-Hydroxy-3-(2,2-dimethyl-3-hydroxypropylamino)propoxy]-3-methyl-indolinon
4-[2-Hydroxy-3-(1-methyl-3-hydroxypropylamino)propoxy]-3,3-dimethyl-indolinon
4-[2-Hydroxy-3-(1,1-dimethyl-3-hydroxypropylamino)propoxy]-3,3-dimethyl-indolinon
4-[2-Hydroxy-3-(1,3-dimethyl-3-hydroxypropylamino)propoxy]-3,3-dimethyl-indolinon
5-[2-Hydroxy-3-(1,3-dimethyl-3-hydroxypropylamino)propoxy]-3,3-dimethyl-indolinon
4-[2-Hydroxy-3-(2,2-dimethyl-3-hydroxypropylamino)propoxy]-3,3-dimethyl-indolinon
4-[2-Hydroxy-3-(1-methyl-3-hydroxypropylamino)propoxy]-3-(pyrazol-5-yl)methylen-indolinon
4-[2-Hydroxy-3-(1,1-dimethyl-3-hydroxypropylamino)propoxy]-3-(pyrazol-5-yl)methylen-indolinon
4-[2-Hydroxy-3-(1,3-dimethyl-3-hydroxypropylamino)propoxy]-3-(pyrazol-5-yl)methylen-indolinon
4-[2-Hydroxy-3-(2,2-dimethyl-3-hydroxypropylamino)propoxy]-3-(pyrazol-5-yl)methylen-indolinon
5-[2-Hydroxy-3-(2,2-dimethyl-3-hydroxypropylamino)propoxy]-3-(pyrazol-5-yl)methylen-indolinon

Beispiel 1 (Zwischenprodukte der Formel VII, nicht beansprucht)

4-[2-Hydroxy-3-(1-methyl-3-nitratopropylamino)propoxy]-3-indolinon-benzoat

4.1 g 4-(2,3-Epoxy-propoxy)-indolinon (Lit.: P 33 10 891.9) werden in 100 ml Methanol suspendiert und mit 14.6 g 1-Methyl-3-nitratopropylamin versetzt. Man ruehrt 3 d bei Raumtemperatur, engt im Vacuum ein, nimmt den Rueckstand in Essigester auf und extrahiert mehrmals mit Wasser. Nach Trocknen mit $Na_2SO_4$ wird abgesaugt und das Filtrat mit der berechneten Benzosaeure versetzt, im Vacuum eingeengt und ueber eine Kieselgelsaeule mit Methylenchlorid/Methanol 95/5 gereinigt. Das Eluat wird im Vacuum eingeengt, mit wenig Essigester aufgenommen und abgesaugt. Es verbleiben 3.9 g der Titelverbindung als Benzoat von Schmp. 133—134°C, d.s. 42% d.Th..

In analoger Weise zu Beispiel 1 erhaelt man:

In analoger Weise zu Beispiel 1 erhaelt man:

| Bezeichnung | Ausbeute [%] | Mp [°C] Lösungsm. |
|---|---|---|
| a) 4-[2-Hydroxy-3-(1,1-dimethyl-3-nitratopropyl-amino)propoxy]indolinon-hemifumarat<br><br>aus<br><br>4-(2,3-Epoxy-propoxy)indolinon und 1,1-Dimethyl-3-nitratopropylamin | 10 | 134-136 Isopro-panol/ Wasser |
| b) 4-[2-Hydroxy-3-(1,3-dimethyl-3-nitratopropyl-amino)propoxy]indolinon-fumarat<br><br>aus<br><br>4-(2,3-Epoxy-propoxy)indolinon und 1,3-Dimethyl-3-nitratopropylamin | 15 | 149-150 Isopro-panol |
| c) 5-[2-Hydroxy-3-(1-methyl-3-nitratopropylamino)-propoxy]indolinon-fumarat<br><br>aus<br><br>5-(2,3-Epoxy-propoxy)indolinon und 1-Methyl-3-nitratopropylamin | 15 | 100 (Zers.) Ethanol |
| d) 6-[2-Hydroxy-3-(1-methyl-3-nitratopropylamino)-propoxy]indolinon-fumarat<br><br>aus<br><br>6-(2,3-Epoxy-propoxy)indolinon und 1-Methyl-3-nitratopropylamin | 20 | 127-128 Ethanol |
| e) 7-[2-Hydroxy-3-(1-methyl-3-nitratopropyl-amino)propoxy]indolinon-fumarat<br><br>aus<br><br>7-(2,3-Epoxy-propoxy)indolinon und 1-Methyl-3-nitratopropylamin | 18 | 141-142 Ethanol |

Die zur Herstellung der vorgenannten Beispiele (1c, 1d, 1e) benoetigten Zwischenprodukte werden nach den in EP—B 1-00 149 28 und P 33 10 891.9 beschreibenen Methoden hergestellt.

| Bezeichnung | Ausbeute [%] | Mp [°C] Lösungsm. |
|---|---|---|
| 5-(2,3-Epoxy-propoxy)indolinon | 50 | 129-130 Ether |
| 6-(2,3-Epoxy-propoxy)indolinon | 40 | 130-131 Essigester |
| 7-(2,3-Epoxy-propoxy)indolinon | 45 | 183 Essigester |

Beispiel 2

4-[2-Hydroxy-3-(1-methyl-3-nitratopropylamino)propoxy]-3-(pyrazol-5-yl)methylen-indolinon-benzoat

3.7 g 4-(2,3-Epoxy-propoxy)-3-(pyrazol-5-yl)methylen-indolinon (Lit.: P 33 10 891.9) werden mit 100 ml Methanol und 10 g 1-Methyl-3-nitratopropylamin 3 d bei Raumtemperatur geruehrt. Nach Entfernen des Loesungsmittels wird in Essigester geloest und mit Wasser mehrmals ausgeschuettelt. Nach Trocknen ueber $Na_2SO_4$ wird abgesaugt und das Filtrat mit der berechneten Menge Benzosaeure versetzt. Nach Absaugen verbleiben 2.5 g der Titelverbindung vom Schmp. 148—150°C, d.s. 35% d.Th..

In analoger Weise zu Beispiel 2 erhaelt man:

| Bezeichnung | Ausbeute [%] | Mp [°C] Lösungsm. |
|---|---|---|
| a) 4-[2-Hydroxy-3-(1-methyl-3-nitratopropyl-amino)propoxy]-3-(pyrrol-2-yl)methylen-indolinon-hemifumarat<br>aus<br>4-(2,3-Epoxy-propoxy)-3-(pyrrol-2-yl)-methylen-indolinon<br>und<br>1-Methyl-3-nitratopropylamin | 10 | 161-163 Ethanol |
| b) 4-[2-Hydroxy-3-(1-methyl-3-nitratopropyl-amino)propoxy]-3-(pyridin-2-yl)methylen-indolinon-fumarat<br>aus<br>4-(2,3-Epoxy-propoxy)-3-(pyridin-2-yl)-methylen-indolinon<br>und<br>1-Methyl-3-nitratopropylamin | 15 | 153-155 Ethanol |
| c) 4-[2-Hydroxy-3-(1-methyl-3-nitratopropyl-amino)propoxy]-3-benzyliden-indolinon-fumarat<br>aus<br>4-(2,3-Epoxy-propoxy)-3-benzyliden-indolinon<br>und<br>1-Methyl-3-nitratopropylamin | 20 | 155-158 Ethanol |

Beispiel 3 (Zwischen produkt der Formel VII, nicht beansprucht)

4-[2-Hydroxy-3-(1-methyl-3-nitratopropylamino)propoxy]-indolinon-fumarat

28.2 g 2-(2,3-Epoxy-propoxy)-6-nitrophenylessigsaeureethylester (EP—B 1-00 14 928) werden in einem Gemisch aus 140 ml Essigester, 140 ml Ethanol und 30 ml Wasser geloest, mit 3 ml Raney-Nickel versetzt und bei 1 bar Wasserstoffdruck hydriert. Nach Absaugen des Katalysators wird eingeengt, der Rueckstand in 200 ml Ethanol geloest, 20 g 1-Methyl-3-nitratopropylamin zugegeben und 1 d bei Raumtemperatur geruehrt. Nach Zugabe von 30 ml Essigsaeure wird 1 d bei Raumtemperatur geruehrt. Die Loesung wird eingeengt und in 750 ml Wasser und 750 ml Essigester/Ether 1:1 geloest. Die waessrige Phase wird mit $NaHCO_3$ neutralisiert und die Base mit Eissigester extrahiert. Nach Trocknen und Eindampfen verbleiben 11 g Base. In Isopropanol wird mit der berechneten Menge Fumarsaeure das Salz hergestellt. Nach Absaugen verbleiben 10 g der Titelverbindung vom Schmp. 123—125°C, d.s. 20% d.Th..

In analoger Weise koennen durch katalytische Hydrierung und anschliessende Umsetzung mit Aminen und Cyclisierung die folgenden Verbindungen erhalten werden:

| | Bezeichnung | Ausbeute [%] | Mp [°C] Lösungsm. |
|---|---|---|---|
| a) | 4-[2-Hydroxy-3-(1,1-dimethyl-3-nitratopropylamino)propoxy]indolinon-hemifumarat<br>aus<br>2-(2,3-Epoxy-propoxy)-6-nitrophenylessigsaeureethylester<br>und<br>1,1-Dimethyl-3-nitratopropylamin | 15 | 134-136 Isopropanol |
| b) | 4-[2-Hydroxy-3-(1,3-dimethyl-3-nitratopropylamino)propoxy]indolinon-fumarat<br>aus<br>2-(2,3-Epoxy-propoxy)-6-nitrophenylessigsaeureethylester<br>und<br>1,3-Dimethyl-3-nitratopropylamin | 25 | 150 Isopropanol |
| c) | 5-[2-Hydroxy-3-(1-methyl-3-nitratopropylamino)propoxy]indolinon-fumarat<br>aus<br>3-(2,3-Epoxy-propoxy)-6-nitrophenylessigsaeureethylester<br>und<br>1-Methyl-3-nitratopropylamin | 20 | 100-103 Ethanol |
| d) | 6-[2-Hydroxy-3-(1-methyl-3-nitratopropylamino)propoxy]indolinon-fumarat<br>aus<br>4-(2,3-Epoxy-propoxy)-6-nitrophenylessigsaeureethylester<br>und<br>1-Methyl-3-nitratopropylamin | 20 | 128-129 Ethanol |

Beispiel 3 (Fortsetzung)

| Bezeichnung | Ausbeute [%] | Mp [°C] Lösungsm. |
| --- | --- | --- |
| e) 7-[2-Hydroxy-3-(1-methyl-3-nitratopropyl-amino)propoxy]indolinon-fumarat<br>aus<br>5-(2,3-Epoxy-propoxy)-6-nitrophenylessig-saeureethylester<br>und<br>1-Methyl-3-nitratopropylamin | 25 | 141-142 Ethanol |

Beispiel 4

4-[2-Hydroxy-3-(1-methyl-3-nitratopropylamino)propoxy]-3-(pyrrol-2-yl)methylen-indolinon-hemifumarat

5.5 g 4-[2-Hydroxy-3-(1-methyl-3-nitratopropylamino)propoxy]-indolinon (Beispiel 1) werden in 100 ml Ethanol mit 3.4 g Pyrrol-2-aldehyd und 5 ml Triethylamin 2 d bei Raumtemperatur geruehrt. Nach Entfernen des Loesungsmittels im Vacuum wird der Rueckstand in Essigester und verduennter Milchsaeure geloest. Die waessrige Phase wird mit Kaliumcarbonat alkalisch gestellt und mit Essigester extrahiert. Nach Reinigen ueber eine Kieselgelsaeule mit Methylenchlorid:Methanol 97:3 wird in Ethanol das Hemifumarat hergestellt. Es verbleiben nach Absaugen 1.6 g der Titelverbindung von Schmp. 161—163°C, d.s. 20% d.Th..

In analoger Weise erhaelt man:

| Bezeichnung | Ausbeute [%] | Mp [°C] Lösungsm. |
|---|---|---|
| a) 4-[2-Hydroxy-3-(1-methyl-3-nitratopropyl-amino)propoxy]-3-(furan-2-yl)methylen-indolinon-fumarat<br>aus<br>4-[2-Hydroxy-3-(1-methyl-3-nitratopropyl-amino)propoxy]indolinon (Beisp. 1)<br>und<br>Furan-2-aldehyd | 35 | 155-158<br>Ethanol |
| b) 4-[2-Hydroxy-3-(1-methyl-3-nitratopropyl-amino)propoxy]-3-(thiophen-2-yl)methylen-indolinon - fumarat<br>aus<br>4-[2-Hydroxy-3-(1-methyl-3-nitratopropyl-amino)propoxy]indolinon (Beisp. 1)<br>und<br>Thiophen-2-aldehyd | 30 | 152-153<br>Methanol |
| c) 4-[2-Hydroxy-3-(1-methyl-3-nitratopropylamino)propoxy]-3-(pyrazol-5-yl)methylen-indolinon-fumarat<br>aus<br>4-[2-Hydroxy-3-(1-methyl-3-nitratopropyl-amino)propoxy]indolinon (Beisp. 1)<br>und<br>Pyrazol-5-aldehyd | 25 | 168-170<br>Methanol |
| d) 4-[2-Hydroxy-3-(1-methyl-3-nitratopropyl-amino)propoxy]-3-(1,2,4-triazol-3-yl)-methylen-indolinon-fumarat<br>aus<br>4-[2-Hydroxy-3-(1-methyl-3-nitratopropyl-amino)propoxy]indolinon (Beisp. 1)<br>und<br>1,2,4-Triazol-3-aldehyd | 40 | 174<br>Methanol |

Beispiel 4 (Fortsetzung)

| Bezeichnung | Ausbeute [%] | Mp [°C] Lösungsm. |
|---|---|---|
| e) 4-[2-Hydroxy-3-(1-methyl-3-nitratopropylamino)propoxy]-3-(pyridin-2-yl)methylen-indolinon-fumarat<br>aus<br>4-[2-Hydroxy-3-(1-methyl-3-nitratopropylamino)propoxy]indolinon (Beisp. 1)<br>und<br>Pyridin-2-aldehyd | 15 | 153-155 Ethanol |
| f) 4-[2-Hydroxy-3-(1-methyl-3-nitratopropylamino)propoxy]-3-(uracil-4-yl)methylen-indolinon-acetat<br>aus<br>4-[2-Hydroxy-3-(1-methyl-3-nitratopropylamino)propoxy]indolinon (Beisp. 1)<br>und<br>Uracil-4-aldehyd | 15 | 190 Methanol |
| g) 4-[2-Hydroxy-3-(1,1-dimethyl-3-nitrato-propylamino)propoxy]-3-(pyrazol-5-yl)-methylen-indolinon-hemifumarat<br>aus<br>4-[2-Hydroxy-3-(1,1-dimethyl-3-nitrato-propylamino)propoxy]indolinon (Beisp. 1 a)<br>und<br>Pyrazol-5-aldehyd | 25 | 143-145 Methanol |
| h) 4-[2-Hydroxy-3-(1,3-dimethyl-3-nitrato-propylamino)propoxy]-3-(pyrazol-5-yl)-methylen-indolinon-fumarat<br>aus<br>4-[2-Hydroxy-3-(1,3-dimethyl-3-nitrato-propylamino)propoxy]indolinon (Beisp. 1 b)<br>und<br>Pyrazol-5-aldehyd | 30 | 70 Zers. Isopropanol |

Beispiel 4 (Fortsetzung)

| Bezeichnung | Ausbeute [%] | Mp [°C] Lösungsm. |
|---|---|---|
| i) 4-[2-Hydroxy-3-(1-methyl-3-nitratopropylamino)propoxy]-3-benzyliden-indolinon-fumarat<br>aus<br>4-[2-Hydroxy-3-(1-methyl-3-nitratopropylamino)propoxy]indolinon (Beisp. 1)<br>und<br>Benzaldehyd | 25 | 155-158 Ethanol |
| j) 4-[2-Hydroxy-3-(1-methyl-3-nitratopropylamino)propoxy]-3-(4-cyano-benzyliden)-indolinon - hemifumarat<br>aus<br>4-[2-Hydroxy-3-(1-methyl-3-nitratopropylamino)propoxy]indolinon (Beisp. 1)<br>und<br>4-Cyan-benzaldehyd | 20 | 155-157 Methanol |
| k) 4-[2-Hydroxy-3-(2,2-dimethyl-3-nitratopropylamino)propoxy]-3-(3-methyl-pyrazol-5-yl)-methylen-indolinon<br>aus<br>4-[2-Hydroxy-3-(2,2-dimethyl-3-nitratopropylamino)propoxy]indolinon<br>und<br>3-Methyl-pyrazol-5-aldehyd | 80 | 150-151 Methanol |

Beispiel 5

4-[2-Hydroxy-3-(1-methyl-3-nitratopropylamino)propoxy]-indolinon fumarat (zwischen produkt der Formel VII, nicht beansprucht)

2,94 g 4-[2-Hydroxy-3-(1-methyl-3-hydroxypropylamino)-propoxy]-indolinon werden in 100 ml Acetonitril bei 30°C unter Rühren mit einer Mischung aus 1,16 ml Acetanhydrid, 0,52 ml rauchender Salpetersäure in 20 ml Acetonitril versetzt. Nach 3 h Stunden Rühren bei −30°C wird die Reaktionsmischung in 300 g Eiswasser eingerührt, mit 1 N Natronlauge auf pH 8 eingestellt und 2 h mit Essigester bei 5°C gerührt. Dann wird auf pH 10 eingestellt und nach 30 Min. die organische Phase abgetrennt. Nach Trocknen und Zugabe der berechneten Menge Fumarsäure erhält man 0,9 g der Titelverbindung. Fp: 122—124°C, Ausb. 20% d.Th.

Die oben genannte Hydroxyverbindung erhält man aus
2-(2,3-Epoxy-propoxy)-6-nitrophenylessigsäureethylester und
1-Methyl-3-hydroxypropylamin
Fp 112—115°C, aus Eissigester; Ausbeute 50% d.Th..

**Patentansprüche**

1. Oxindol-Derivate der allgemeinen Formel I

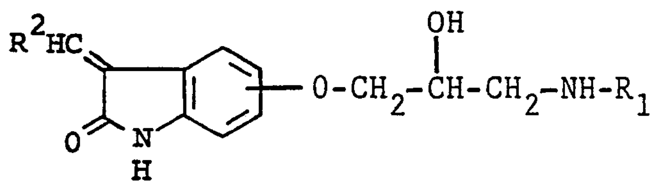

(I)

in der

$R_1$ eine $C_2$—$C_{10}$-Nitratoalkylgruppe,

$R_2$ einen geradkettigen oder verzweigten $C_1$—$C_6$-Alkylrest, einen Cyclopentyl- oder Cyclohexylrest, oder eine unsubstituierte oder durch Halogen-, $C_1$—$C_6$-Alkyl, $C_1$—$C_6$-Hydroxyalkyl-, Hydroxy-, $C_1$—$C_6$-Alkoxy-, $C_2$—$C_4$-Alkenyl-, $C_2$—$C_4$-Alkenyloxy, $C_2$—$C_4$-Alkinyl-, Amino-, $C_1$—$C_4$-Alkylamino-, $C_2$—$C_8$-Dialkylamino-, Aminocarbonyl-, $C_1$—$C_4$-Alkylaminocarbonyl-, $C_2$—$C_8$-Dialkylaminocarbonyl-, Cyano-, $C_2$—$C_4$-Alkanoyl-, Aminosulfonyl-, $C_1$—$C_4$-Alkylaminosulfonyl-, $C_2$—$C_8$-Dialkylaminosulfonyl-, Carboxyl-, $C_1$—$C_6$-Alkoxycarbonyl-, $C_1$—$C_6$-Alkylthio-, $C_1$-$C_6$-Alkylsulfinyl-, $C_1$—$C_6$-Alkylsulfonyl-, $C_2$—$C_4$-Alkanoylamido-, $C_1$—$C_4$-Alkylsulfonylamido- oder Nitrogruppen oder durch eine $C_1$—$C_2$-Alkyendioxygruppe substituierte Phenyl-, Furyl-, Thiophenyl, Pyrrolyl-, Pyrazolyl-, Imidazolyl-, Triazolyl-, Tetrazolyl-, Oxoimidazolinyl-, Pyridinyl-, Pyrimidinyl-, Uracilyl-, Indolyl-, Indazolyl- oder Dihydropyranylgruppe darstellt, deren optisch aktive Formen sowie deren pharmakologisch unbedenkliche Salze.

2. Verbindungen gemäß Anspruch 1, in denen $R_1$ eine Nitratoethyl-, Nitratopropyl-, Nitratobutyl-, Nitratopentyl-, Nitratohexyl-, 1-Methyl-2-nitratoethyl-, 1-Methyl-3-nitratopropyl-, 1,1-Dimethyl-3-nitratopropyl-, 1,3-Dimethyl-3-nitratopropyl- oder 2,2-Dimethyl-3-nitratopropylgruppe bedeutet.

3. Verbindungen gemäß Anspruch 1 oder 2, in denen $R_1$ die 1-Methyl-3-nitratopropyl-, 1,1-Dimethyl-3-nitratopropyl- oder die 1,3-Dimethylnitratopropyl-Gruppe darstellt.

4. Verbindungen gemäß Anspruch 1, 2 oder 3, in der $R_2$ einen Phenyl-Rest, der durch Halogen, Methyl, Hydroxymethyl, Hydroxy, Methoxy, Allyloxy, Amino, Dimethylamino, Acetylamino, Methylsulfonylamino, Acetyl, Carboxyl, Methoxycarbonyl, Carbamoyl, Dimethylcarbamoyl, Nitril, Nitro, Dimethylsulfonamido, Methylsulfenyl oder Dioxymethylen substituiert sein kann.

5. Verbindungen gemäß Anspruch 4, in denen $R_2$ einen Furanyl-, Thiophenyl, Pyrrolyl-, Pyrazolyl-, Imidazolyl-, Triazolyl-, Tetrazolyl-, Oxoimidazolinyl-, Pyridinyl-, Pyrimidinyl-, Uracilyl-, Indolyl-, Indazolyl- und Dihydropyranyl-Rest bedeuten, wobei diese Hetaryle durch Methyl oder Methylaminosulfonyl substituiert sein können.

6. Verfahren zur Herstellung von Oxindol-Derivaten wie in den Ansprüchen 1—5 angegeben, dadurch gekennzeichnet, daß man in an sich bekannter Weise

a) eine Verbindung der allgemeinen Formel II

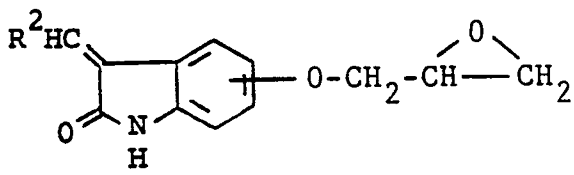

(II)

mit einer Verbindung der allgemeinen Formel III

$$H_2N—R_1 \quad (III)$$

wobei $R_1$ und $R^2$ die zu den Ansprüchen 1—5 angegebene Bedeutung haben, umsetzt oder

b) eine Verbindung der allgemeinen Formel IIa

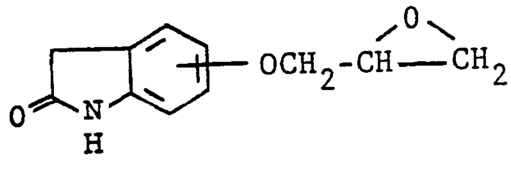

(IIa)

Y Wasserstoff bedeuten, mit einer Verbindung der allgemeinen Formel IV

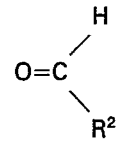

(IV)

in welcher $R^2$ die oben angegebenen Bedeutung hat kondensiert und anschließend mit einer Verbindung der allgemeinen Formel III
umsetz oder

c) eine Verbindung der allgemeinen Formel V

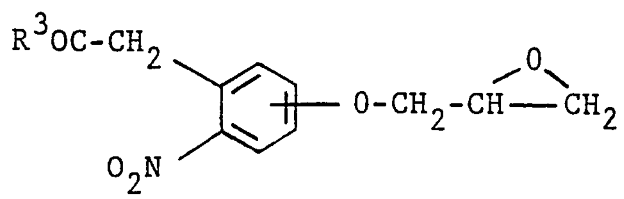

(V)

in welcher $R_3$ eine abspaltbare Gruppe darstellt, reduziert, mit einer Verbindung der allgemeinen Formel III unsetzt und
die dabei erhaltene Verbindung der allgemeinen Formel VI

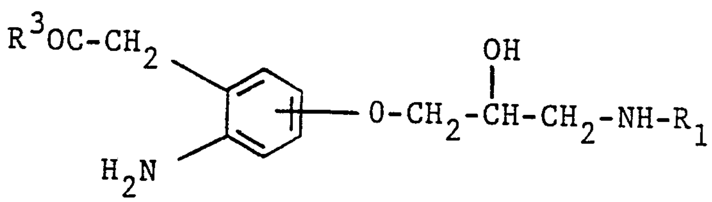

(VI)

in welcher $R_1$ und $R_3$ die oben angegebenen Bedeutung haben, cyclisiert oder

d) eine Verbindung der allgemeinen Formel VII

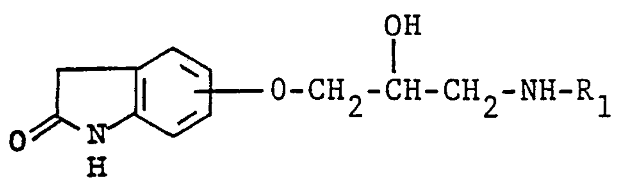

(VII)

in welcher $R_1$, die zu den angegebene Bedeutung hat, mit einer Verbindung der allgemeinen Formel IV umsetzt oder

e) eine Verbindung der allgemeinen Formel VIII

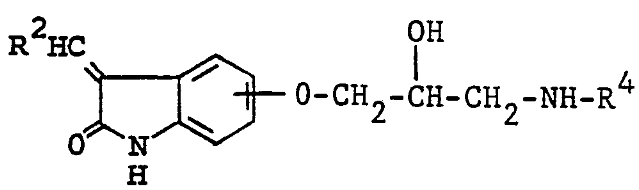

(VIII)

in welcher $R^2$ die zu den angegebene Bedeutung hat, und $R_4$ eine $C_2$—$C_{10}$ Hydroxyalkylgruppe darstellt, mit Salpetersäure oder einem reaktiven Derivat davon, umsetzt, und gewünschtenfalls die erhaltenen Verbindungen in ein pharmakoligisch unbedenkliches Salz überführt.

7. Arzneimittel enthaltend eine Verbindung gemäß Anspruch 1—5 sowie übliche Träger- und Hilfsstoffe.

8. Verwendung von Verbindungen gemäß Anspruch 1—5 zur Herstellung von Arzneimitteln zur Behandlung und Prophylaxe von Herz- und Kreislauferkrankungen.

9. Verbindungen der allgemeinen Formel VI

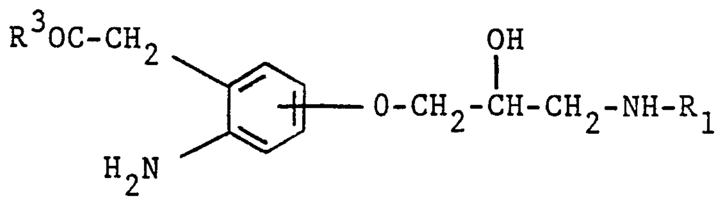

(VI)

in der

$R_1$ eine $C_2$—$C_{10}$-Nitratoalkylgruppe und

$R_3$ Amino, Hydroxy, Imidazolyl- oder $C_1$—$C_6$-Alkoxy bedeutet.

10. Verbindungen der allgemeinen Formel VIII und VIIIa

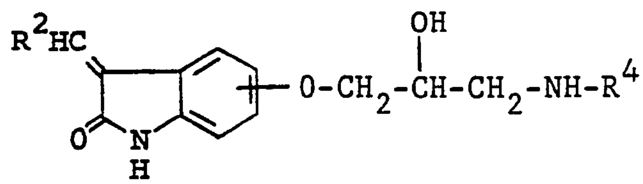

(VIII)

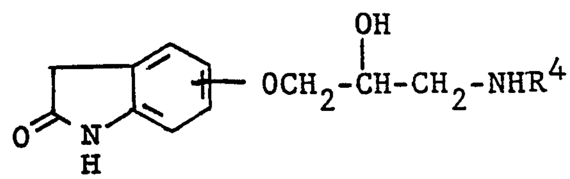

(VIIIa)

in der

$R^2$ die an Anspruch 1 angebene Bedeutung bericht und

$R_4$ eine $C_2$—$C_{10}$-Hydroxyalkylgruppe ist.

11. Verwendung von Verbindungen gemäß Anspruch 8 oder 9 zur Herstellung von Verbindungen der Formel I wie in den Ansprüchen 1—5 angegeben.

## Revendications

1. Dérivés d'oxindole de formule générale I

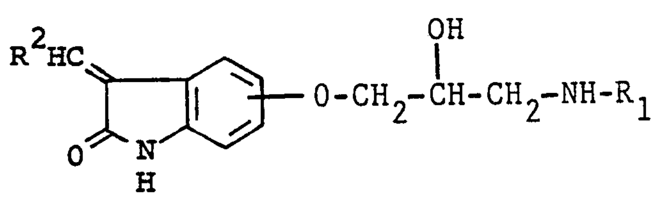

(I)

dans laquelle

$R^1$ représente un groupe nitratoalkyle($C_2$—$C_{10}$), un groupe alkyle ($C_1$—$C_6$) à chaîne droite ou ramifiée, un groupe cyclopentyle ou cyclohexyle ou un groupe phényle, furyle, thiophényle, pyrrolyle, pyrazolyle, imidazolyle, triazolyle, tétrazolyle, oxoimidazolinyle, pyridinyle, pyrimidinyle, uracyle, indolyle, indazolyle ou dihydropyranyle, non substitué ou substitué par un groupe halogène, alkyle($C_1$—$C_6$), hydroxyalkyle-($C_1$—$C_6$), hydroxy, alcoxy($C_1$—$C_6$), alcényle($C_2$—$C_4$), alcényl($C_2$—$C_4$)oxy, alcinyl($C_2$—$C_4$), amino, alkyl-($C_1$—$C_4$)amino, dialkyl($C_2$—$C_8$)amino, aminocarbonyle, alkyl($C_1$—$C_4$)aminocarbonyle, dialkyl($C_2$—$C_8$)-aminocarbonyle, cyano, alcanoyle($C_2$—$C_4$), aminosulfonyle, alkyl($C_1$—$C_4$)aminosulfonyle, dialkyl($C_2$—$C_8$)-aminosulfonyle, carboxyle, alcoxy($C_1$—$C_6$)carbonyle, alkyl($C_1$—$C_6$)thio, alkyl($C_1$—$C_6$)sulfinyle, alkyl($C_1$—$C_6$)-sulfonyle, alcanoyl($C_2$—$C_4$)amido, alkyl($C_1$—$C_4$)sulfonylamino ou nitro, ou par un groupe alkylène($C_1$—$C_2$)-dioxy,

leurs formes optiquement actives ainsi que leurs sels pharmaceutiquement acceptables.

2. Composés selon la revendication 1, dans lesquels $R_1$ représente un groupe nitratoéthyle, nitratopropyle, nitratobutyle, nitratopentyle, nitratohexyle, 1-méthylnitratoéthyle, 1-méthyl-3-nitratopropyle, 1,1-diméthyl-3-nitratopropyle, 1,3-diméthyl-3-nitratopropyle ou 2,2-diméthyl-3-nitratopropyle.

3. Composés selon la revendication 1 ou 2, dans lesquels $R_1$ représente le groupe 1-méthyl-3-nitratopropyle, 1,1-diméthyl-3-nitratopropyle ou 1,3-diméthylnitratopropyle.

4. Composés selon la revendication 1, 2 ou 3, dans lesquels $R_2$ représente un groupe phényle, qui peut être substitué par un groupe halogène, méthyle, hydroxyméthyle, hydroxy, méthoxy, allyloxy, amino, diméthylamino, acétylamino, méthylsulfonylamino, acétyle, carboxyle, méthoxycarbonyle, carbamoyle, diméthylcarbamoyle, nitrile, nitro, diméthylsulfonamido, méthylsulfényle ou dioxyméthylène.

5. Composés selon la revendication 4, dans lesquels $R_2$ représente un groupe furanyle, thiophényle, pyrrolyle, pyrazolyle, imidazolyle, triazolyle, tétrazolyle, oxoimidazolinyle, pyridinyle, pyrimidinyle, uracyle, indolyle, indazolyle et dihydropyranyle, ces groupes hétéroaryle pouvant être substitués par un groupe méthyle ou méthylaminosulfonyle.

6. Procédé pour la préparation des dérivés d'oxindole tels que définis dans les revendications 1—5, caractérisé en ce que, de manière connue en soi,

a) on fait réagir un composé de formule générale II

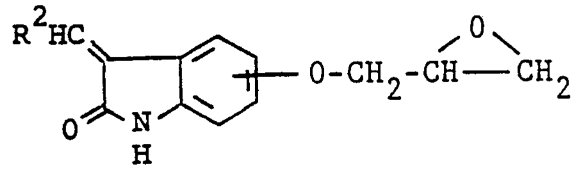

(II)

avec un composé de formule générale III

$$H_2N—R_1 \qquad (III)$$

où $R_1$ et $R_2$ ont les significations indiquées dans les revendications 1—5, ou

b) on condense un composé de formule générale IIa

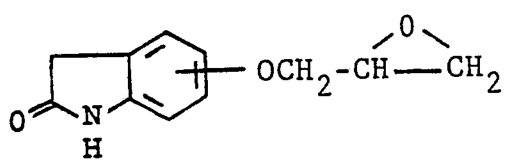

(IIa)

avec un composé de formule générale IV

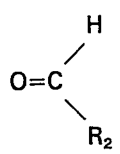

(IV)

où $R_2$ a la signification mentionnée ci-dessus, et ensuite, on fait réagir avec un composé de formulé générale III ou

c) on réduit un composé de formule générale V

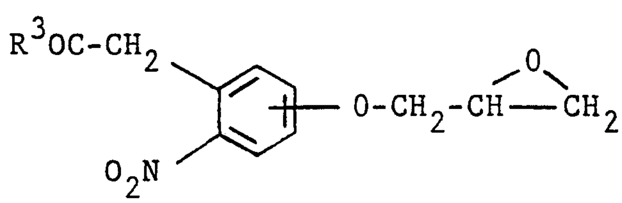

(V)

dans lequel $R_3$ représente un groupe séparable, on le fait réagir avec un composé de formule générale III et on cyclise le compose ainsi obtenu, de formule générale VI

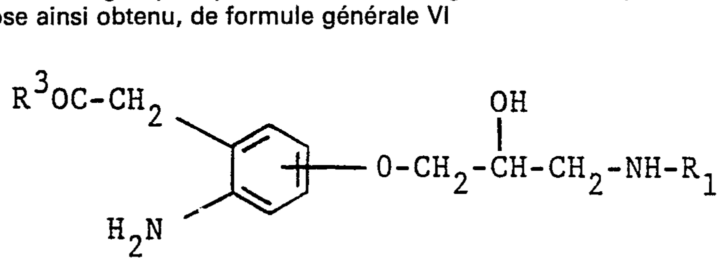

(VI)

où $R_1$ et $R_3$ ont les significations mentionnées, ou

d) un fait réagir un composé de formule générale VII

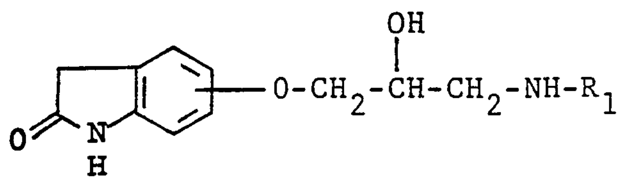

(VII)

où $R_1$ a la signification indiquée dans les revendications 1—5, avec un composé de formule générale IV ou

e) on fait réagir un composé de formule générale VIII

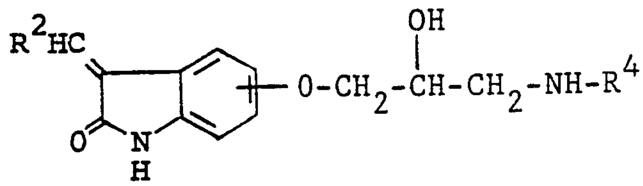

(VIII)

où $R_2$ a la signification indiquée dans les revendications 1—5 , et $R_4$ représente un groupe hydroxyalkyle-($C_2$—$C_{10}$), avec l'acide nitrique ou un de ses dérivés réactifs, et éventuellement, on transforme les composés obtenus en leurs sels pharmaceutiquement acceptables.

7. Médicament contenant un composé selon les revendications 1—5, ainsi que des véhicules et adjuvants usuels.

8. Utilisation des composés selon les revendications 1—5 pour la préparation de médicaments destinés au traitement et à la prévention de maladies cardiovasculaires.

9. Composés de formule générale VI

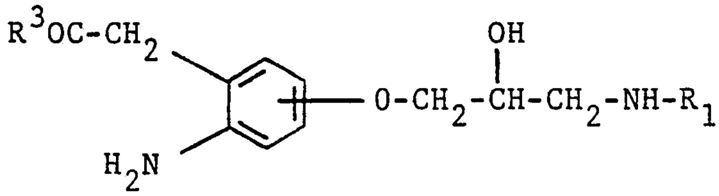

(VI)

où

$R_1$ représente un groupe nitratoalkyle($C_2$—$C_{10}$), et

$R_2$ représente un groupe amino, hydroxy, imidazolyle ou alcoxy($C_1$—$C_6$).

10. Composés de formules générales VIII et VIIIa

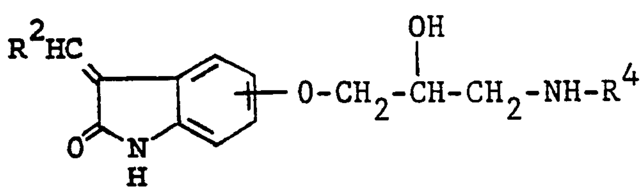

(VIII)

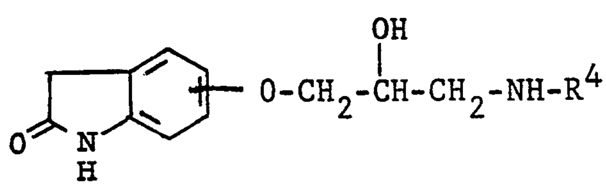

(VIIIa)

où $R_2$ a la signification indiquée dans la revendication 1, et $R_4$ représente un groupe hydroxyalkyle($C_2$—$C_{10}$).

11. Utilisation des composés selon la revendication 8 ou 9, pour la préparation des composés de formule I, tels que définis dans les revendications 1—5.

**Claims**

1. Oxindole derivatives of the general formula I

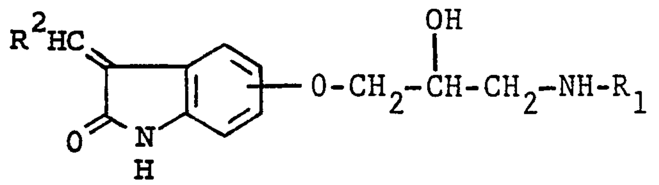

(I)

in which $R_1$ represents a $C_2$—$C_{10}$-nitratoalkyl group, $R_2$ a straight-chained or branched $C_1$—$C_6$-alkyl radical, a cyclopentyl or cyclohexyl radical or a phenyl, furyl, thiophenyl, pyrrolyl, pyrazolyl, imidazolyl, triazolyl, tetrazolyl, oxoimidazolinyl, pyridinyl, pyrimidinyl, uracilyl, indolyl, indazolyl or dihydropyranyl group unsubstituted or substituted by halogen, $C_1$—$C_6$-alkyl, $C_1$—$C_6$-hydroxyalkyl, hydroxyl, $C_1$—$C_6$-alkoxy, $C_2$—$C_4$-alkenyl, $C_2$—$C_4$-alkenyloxy, $C_2$—$C_4$-alkynyl, amino, $C_1$—$C_4$-alkylamino, $C_2$—$C_8$-dialkylamino, aminocarbonyl, $C_1$—$C_4$-alkylaminocarbonyl, $C_2$—$C_8$-dialkylaminocarbonyl, cyano, $C_2$—$C_4$-alkanoyl, aminosulphonyl, $C_1$—$C_4$-alkylaminosulphonyl, $C_2$—$C_8$-dialkylaminosulphonyl, carboxyl, $C_1$—$C_6$-alkoxycarbonyl, $C_1$—$C_6$-alkylthio, $C_1$—$C_6$-alkylsulphinyl, $C_1$—$C_6$-alkylsulphonyl, $C_2$—$C_4$-alkanoylamido, $C_1$—$C_4$-alkylsulphonylamido or nitro group or by a $C_1$—$C_2$-alkylenedioxy group, their optically-active forms, as well as their pharmacologically acceptable salts.

2. Compounds according to claim 1, in which $R_1$ signifies a nitratoethyl, nitratopropyl, nitratobutyl, nitratopentyl, nitratohexyl, 1-methyl-2-nitratoethyl, 1-methyl-3-nitratohexyl, 1,1-dimethyl-3-nitratopropyl, 1,3-dimethyl-3-nitratopropyl or 2,2-dimethyl-3-nitratopropyl group.

3. Compounds according to claim 1 or 2, in which $R_1$ represents the 1-methyl-3-nitratopropyl, 1,1-dimethyl-3-nitratopropyl or the 1,3-dimethylnitratopropyl group.

4. Compounds according to claim 1, 2 or 3, in which $R_2$ represents a phenyl radical which can be substituted by halogen, methyl, hydroxymethyl, hydroxyl, methoxy, allyloxy, amino, dimethylamino, acetylamino, methylsulphonylamino, acetyl, carboxyl, methoxycarbonyl, carbamoyl, dimethylcarbamoyl, nitrilo, nitro, dimethylsulphonamido, methylsulphenyl or dioxymethylene.

5. Compounds according to claim 4, in which $R_2$ signifies a furanyl, thiophenyl, pyrrolyl, pyrazolyl, imidazolyl, triazolyl, tetrazolyl, oxoimidazolinyl, pyridinyl, pyrimidinyl, uracilyl, indolyl, indazolyl and dihydropyranyl radical, whereby these heteroaryls can be substituted by methyl or methylaminosulphonyl.

6. Process for the preparation of oxindole derivatives as given in claims 1—5, characterised in that, in per se known manner, one

a) reacts a compound of the general formula II

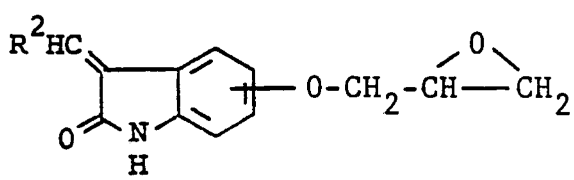

(II)

with a compound of the general formula III

$$H_2N—R_1 \quad (III)$$

whereby $R_1$ and $R_2$ have the meanings given in claims 1—5, or

b) condenses a compound of the general formula IIa

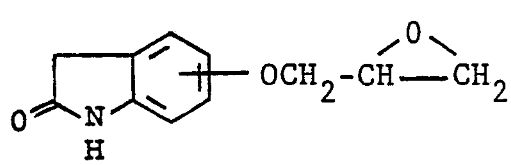

(IIa)

with a compound of the general formula IV

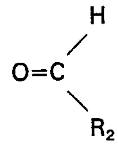

(IV)

in which $R^2$ has the above-given meaning, and subsequently reacts with a compound of the general formula III, or

c) reduces a compound of the general formula V

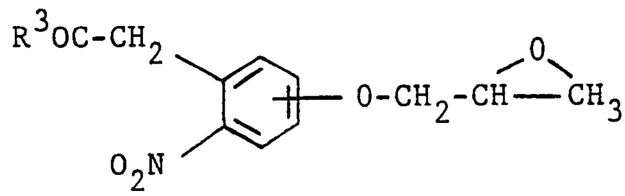

(V)

in which $R^3$ represents a group which can be split off, and reacts with a compound of the general formula III and cyclises the thereby-obtained compound of the general formula VI

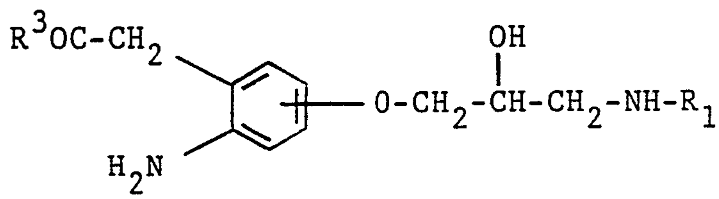

(VI)

in which $R_1$ and $R^3$ have the given meaning, or

d) reacts with a compound of the general formula VII

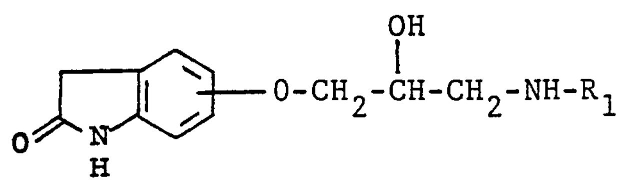

(VII)

in which $R_1$ has the meaning given in claims 1—5, with a compound of the general formula IV, or

e) reacts a compound of the general formula VIII

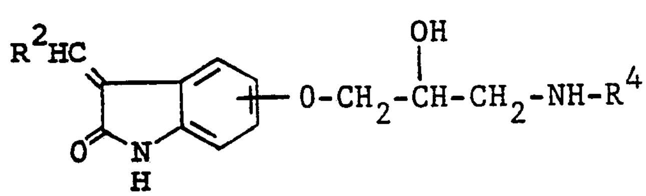

(VIII)

in which $R^2$ has the meaning given in claims 1—5 and $R^4$ represents a $C_2$—$C_{10}$-hydroxyalkyl group, with nitric acid or a reactive derivative thereof and, if desired, converts the compounds obtained into a pharmacologically acceptable salt.

7. Medicaments containing a compound according to claims 1—5, as well as usual carrier and adjuvant materials.

8. Use of compounds according to claims 1—5 for the preparation of medicaments for the treatment and prophylaxis of heart and circulatory diseases.

9. Compounds of the general formula VI

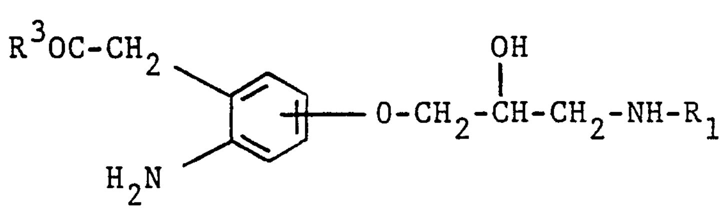

(VI)

in which $R_1$ signifies a $C_2$—$C_{10}$-nitratoalkyl group and $R^3$ amino, hydroxyl, imidazol or $C_1$—$C_6$-alkoxy.

10. Compounds of the general formulae VIII and VIIIa

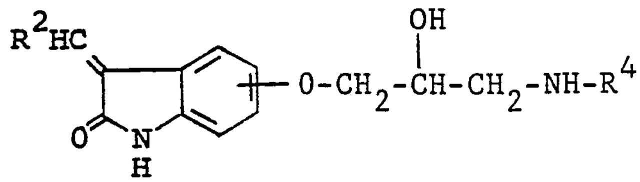

(VIII)

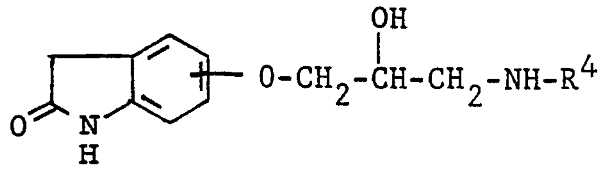

(VIIIa),

in which $R^2$ possesses the meaning given in claim 1 and $R^4$ is a $C_2$—$C_{10}$-hydroxyalkyl group.

11. Use of compounds according to claim 8 or 9 for the preparation of compounds of the formula I as given in claims 1—5.